# EUROPEAN PATENT SPECIFICATION

(11) **EP 0 465 155 B1**
(45) Date of publication and mention of the grant of the patent: **28.02.1996**
(21) Application number: 91305876.4
(22) Date of filing: 28.06.1991
(51) Int. Cl.: C07J 9/00, G02F 1/35, C07J 41/00, C07J 31/00, C07J 43/00

(54) **Non-linear optical material containing steroidal ketone compound**
Nichtlinearer optischer Stoff, welcher ein steroidisches Keton enthält
Matériau optique, non-linéaire, contenant un composé stéroide-ketone

(30) Priority: 29.06.1990 JP 172898/90; 30.11.1990 JP 329466/90
(43) Date of publication of application: 08.01.1992
(73) Proprietor: Hitachi Chemical Co., Ltd., Shinjuku-ku, Tokyo 160 (JP)
(72) Inventor: Kaji, Makoto, Hitachi-shi (JP)
(74) Representative: Cresswell, Thomas Anthony

(56) References cited:
- EP-A- 0 262 672
- DE-A- 2 417 357
- TE-T-
- US-A- 4 150 105
- JOURNAL OF THE CHEMICAL SOCIETY. C no. 11, 1969, LETCHWORTH GB pages 1597 - 1602; E. R. H. JONES ET AL: 'Studies in The Steroid Group. Part LXXIX. Preparation of 2-Aza-3-oxo, 3-Aza-2-oxo,16-Aza-17-oxo, and 17-Aza-16-oxo-5-alpha-androstane, and of 3-Aza-2-oxo-5-alpha-cholestane'
- JOURNAL OF THE CHEMICAL SOCIETY. C. no. 2, 1970, LETCHWORTH GB pages 244 - 250; J. E. BRIDGEMAN ET AL: 'Studies in The Steroid Group. Part LXXX. Preparation of 2- and 16-Oxo, and 3,16- and 2,16-Dioxo-5-alpha-androstane and 2-Oxo-5-alpha-Cholestane'
- JOURNAL OF THE CHEMICAL SOCIETY. C. no. 8, 1970, LETCHWORTH GB pages 1052 - 1055; A.T. DE B. ANDREWS ET AL: 'Hydroxy Steroids. Part XIII. 5-beta-Ergosta-7,22-dien-3-one and Related Compounds'
- COMPTES RENDUS HEBDOMADAIRES DES SEANCES DE L'ACADEMIE DES SCIENCES. SERIE C. vol. 265, no. 17, 23 October 1967, MONTREUIL FR pages 929 - 931; M. MARCEL ET AL: 'Synthèse de Céto-2 Steroides'
- JOURNAL OF THE CHEMICAL SOCIETY. C. no. 4, 1972, LETCHWORTH GB pages 492 - 498; A. S. CLEGG ET AL: 'Studies in The Steroid Group. Part LXXXII. The Preparation of Nine Mono- and Eight Di-oxoandrostanes, 5-alpha-Estran-17-one, and 5-alpha-Pregnane-2,20-dione'
- BULLETIN DE LA SOCIETE CHIMIQUE DE FRANCE. PT 2.no. 1-2, February 1976, PARIS FR pages 255 - 259; J.B. CAZAUX: 'Stéoidopyrazoles et -pyrazolines. I. - Derivés d Arylidène-2 androstanolones-3'
- CHEMICAL ABSTRACTS, vol. 72, no. 11, 16 March 1970, Columbus, Ohio, US; abstract no. 55740, S. HAYASHI ET AL: 'Synthesis of Steroids Having an Arylcyclopropane Ring at Positions 2 and 3' page 468 ;column 1 ;
- OPTICS AND SPECTROSCOPY. vol. 34, no. 2, February 1973, WASHINGTON US pages 150 - 153; B. L. DAVYDOV ET AL: 'Nature of The Absorption Bands and The Origin of The High Nonlinear Dielectric Susceptibility of Molecular Crystals'
- TETRAHEDRON (INCL. TETRAHEDRON REPORTS) vol. 34, no. 8, 1978, OXFORD GB pages 1179 - 1186; J. MUZART ET AL : "Rearrangements Photochimiques d,alpha-Epoxycetones Spiranniques et de Composes Dicarbonyles; Nouvelle Etude de la Photolyse du Benzoyloxy-3, 5-cholestene-2; Photoepimerisation en 5 de la Benzoyl-2, 5alpha-cholestanone-3" page 1183, column 2 - page 1184, column 1

## Description

The present invention relates to a novel steroidal ketone compound which can be used for an device in optical parametric oscillation, higher harmonic wave generation, electro-optical switching, etc., a process for producing the same, a non-linear optical material and a non-linear optical device.

Non-linear optical materials which are expected to play an important role in the optical communication technology and the like exhibit such functions as optical mixing, optical parametric oscillation, higher harmonic wave generation, electro-optical switching, optical rectification and light-light switching, based on the non-linear optical susceptibility of the materials. Previously, inorganic crystals such as those of KH₂PO₄ and NH₄H₂PO₄ have been used as such materials. However, these materials are disadvantageous in that their non-linear optical susceptibility is considerably low for practical use and further they are deliquescent.

Organic non-linear optical materials, which make use of the polarization of the π electron system, have a high non-linear optical susceptibility as compared with prior materials, and also are excellent in high-speed response and damage threshold value, so that research and development thereof have been widely forwarded recently in various fields. Recent results of such efforts are described in detail in, for example, "Nonlinear Optical Properties of Organic and Polymeric Materials" (edited by D.J. Williams, ACS Symposium Series No. 233, published by American Chemical Society, Washington, DC, 1983), "Nonlinear Optical Properties of Organic Molecules and Crystals", Vol. 1 and Vol. 2 (edited by D.S. Chemla and J. Zyss, published by Academic Press, Orlando, FL, 1987), "Nonlinear Optical and Electroactive Polymers" (edited by P.N. Prasad and D.R. Vlrich, published by Prenum Press, New York, 1987) and "Nonlinear Optical Effects in Organic Polymers" (edited by J. Messier, F. Kajar, P.N. Prasad and D.R. Vlrich, published by Klewer Academic Publishers, Dordrecht, The Netherlands, 1989).

However, no material has yet been found which has a large non-linear optical coefficient of such an extent as enables satisfactory attainment of the purpose in low output lasers as semiconductor lasers. Also, the problem of poor thermal stability, which in a shortcoming common to organic materials, has not been solved. Accordingly, further development of novel materials is eagerly desired.

An object of the present invention is to provide an organic non-linear optical material having a high non-linear optical coefficient, a novel steroidal ketone compound which enables the production of said material, a process for producing the same, and a non-linear optical device using the same.

According to the present invention there is provided a non-linear optical material comprising a steroidal ketone of the formula (I) 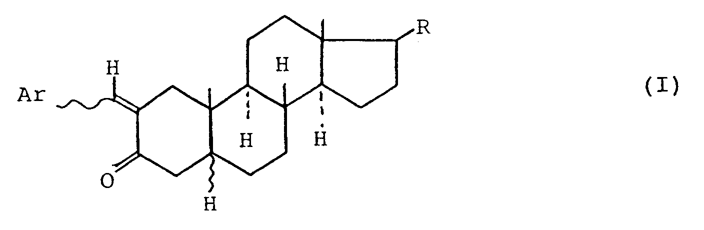
or the formula (II) 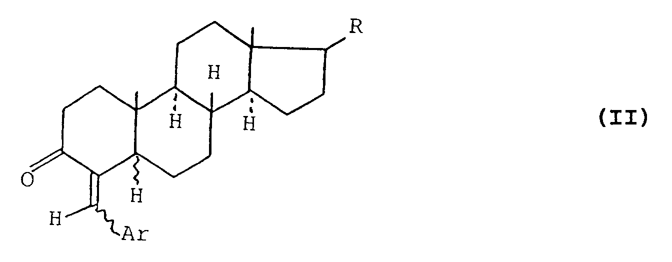
wherein R is a hydrogen atom or straight or branched alkyl group of 1 to 20 carbon atoms; Ar is a group 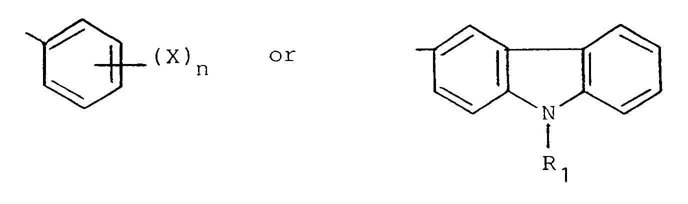
and each X is the same or different and is an amino group, an alkylamino group, a dialkylamino group, an acylated amino group, a hydroxyl group, an alkoxy group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I) or formula (II) is E or Z and the hydrogen in the 5-position has α- or β-configuration.

The invention also provides, as novel compounds, steroidal ketones of the formula (I') 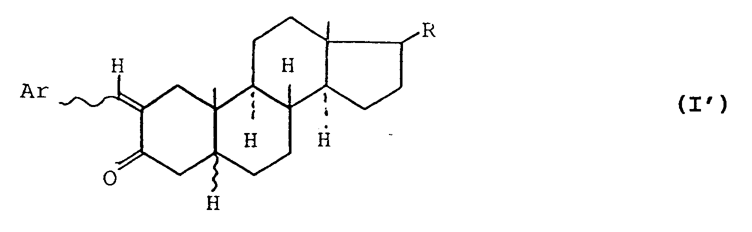
or the formula (II') 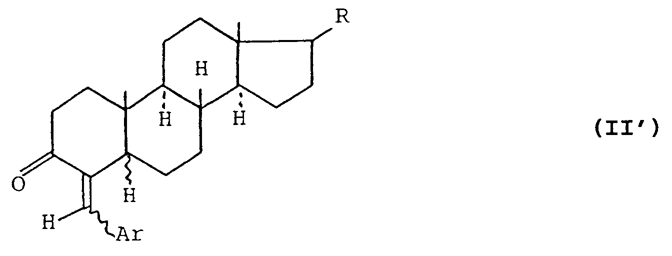
wherein R is a hydrogen atom or straight or branched alkyl group of 1 to 20 carbon atoms; Ar is a group 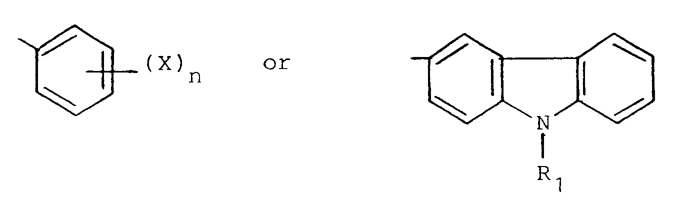
and each X is the same or different and is an amino group, an alkylamino group, a dialkylamino group, an acylated amino group, a hydroxyl group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I') or formula (II') is E or Z and the hydrogen in the 5-position has α- or β-configuration.

The steroidal ketone compounds of the formula (I) or (II) (and (I') or (II')) may be prepared by reacting an aldehyde represented by the formula:

Ar - CHO (V) (or (V'))

wherein Ar is as defined above for formula (I) and (II) or (I') and (II') respectively, with a steroidal ketone compound of the formula: 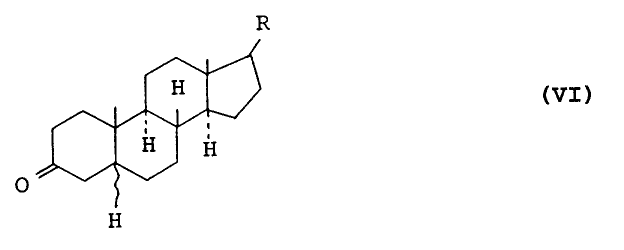
wherein R is as defined above and the bond depicted by a wavy line is R or S .

The present invention further provides a non-linear optical material comprising the steroidal ketone compound of the formula I or II and a non-linear optical element using the non-linear optical material.

### BRIEF DESCRIPTION OF THE DRAWINGS

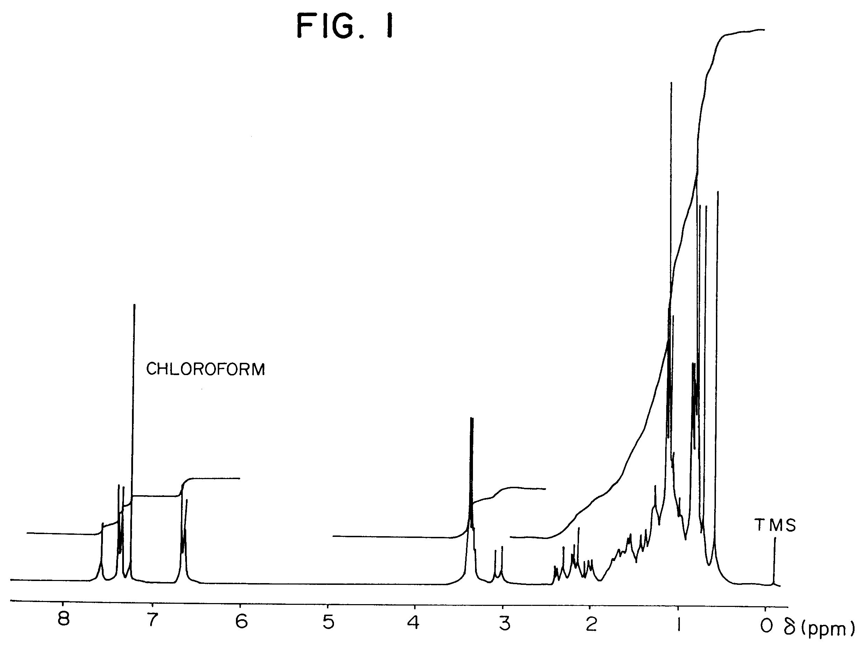

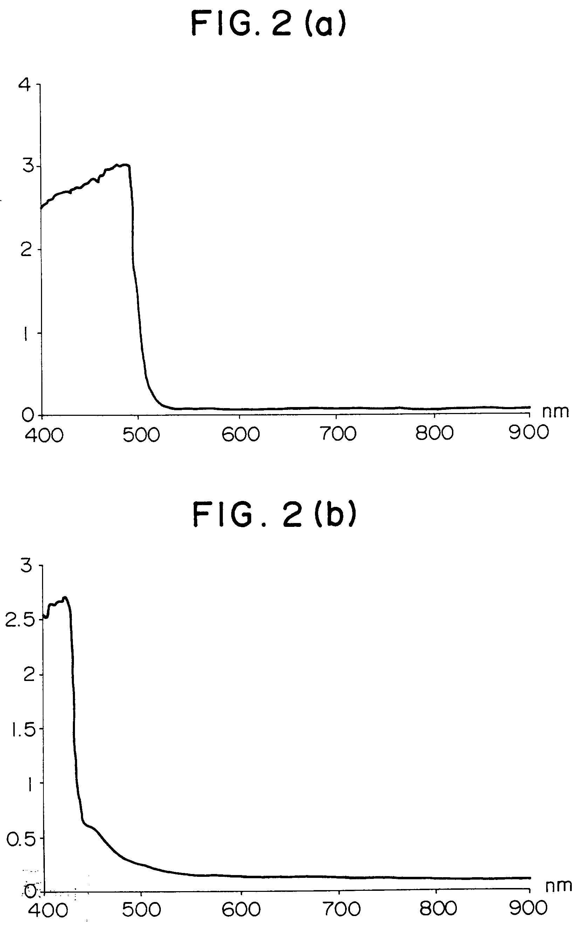

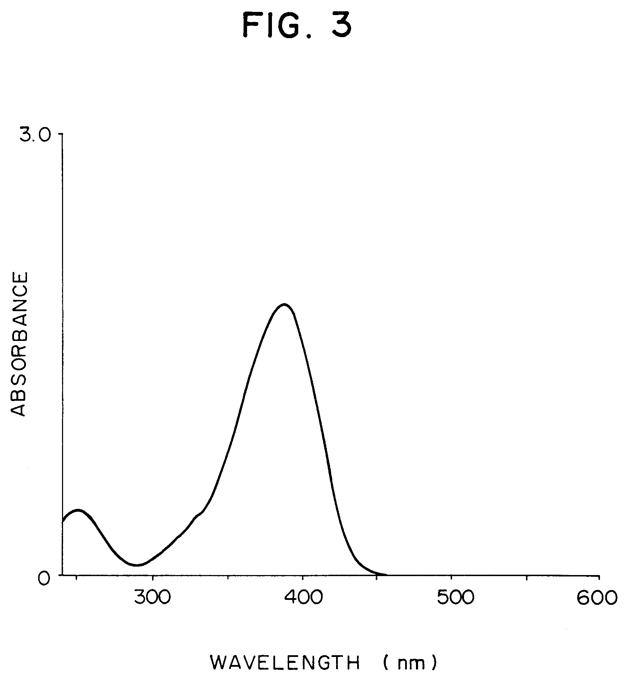

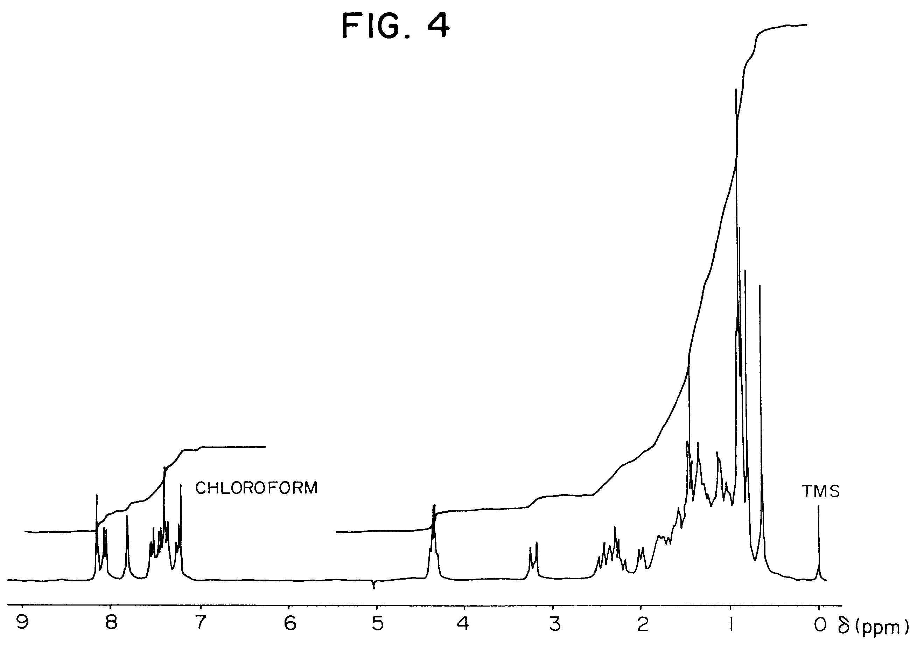

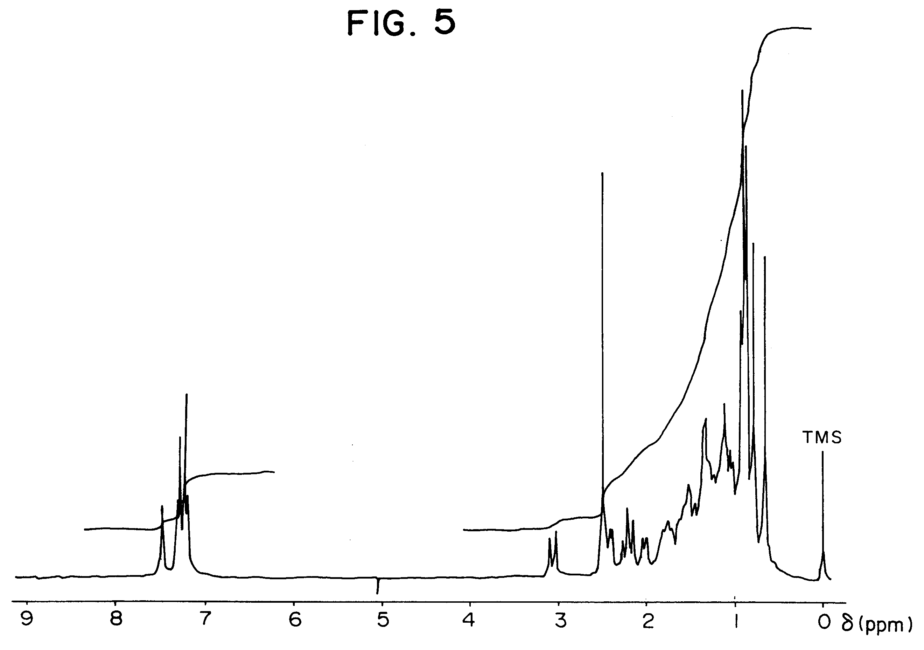

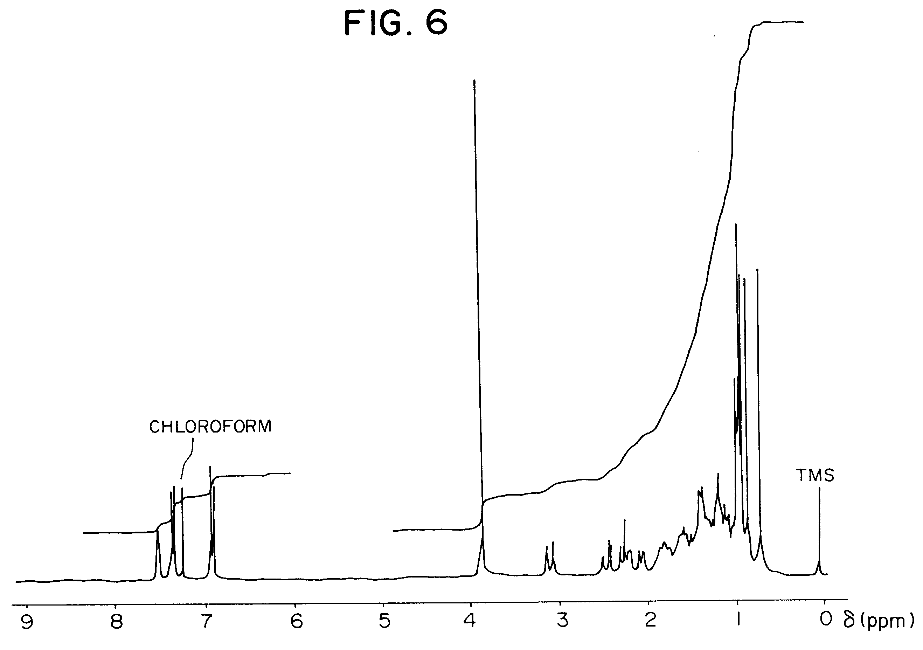

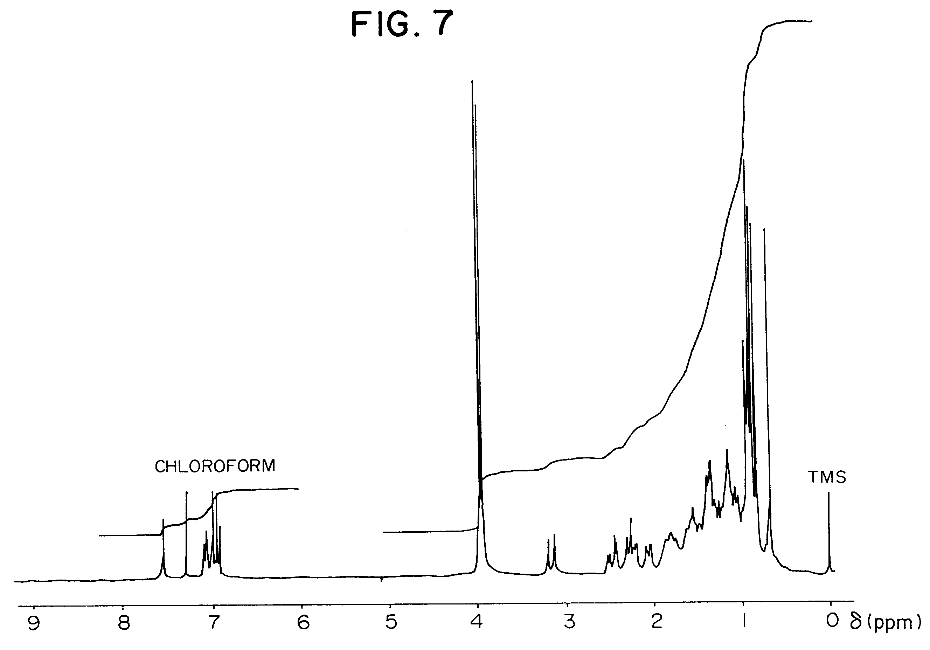

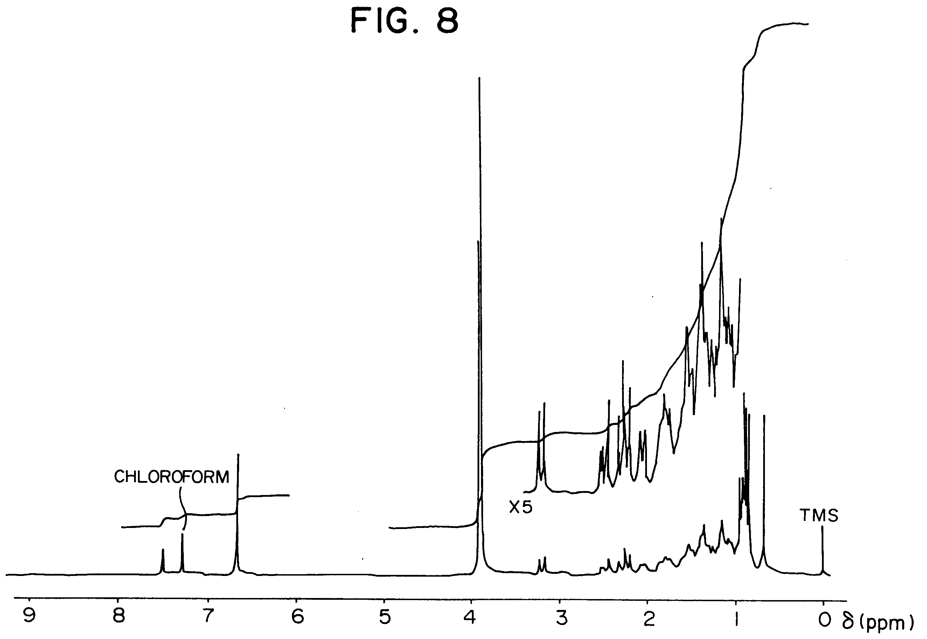

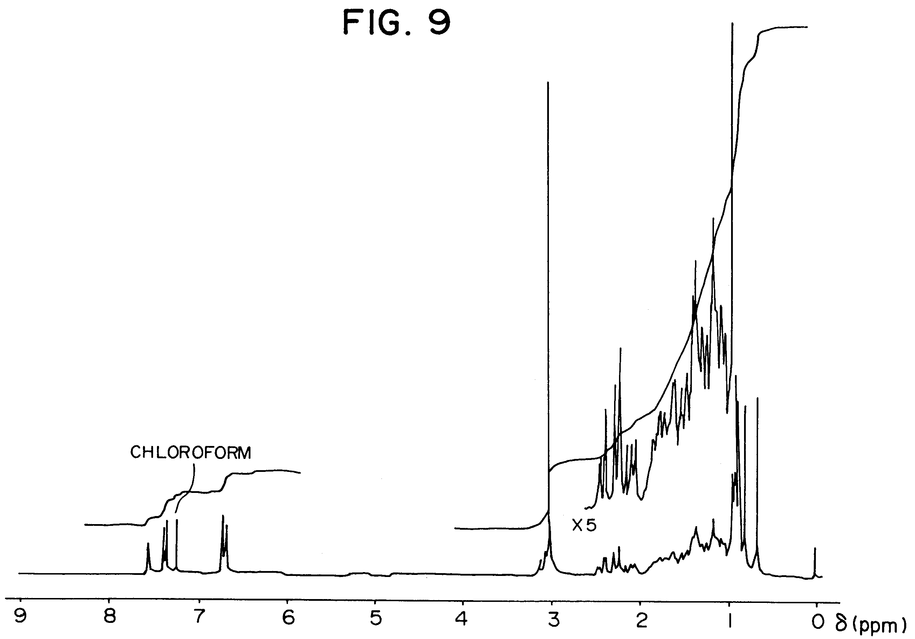

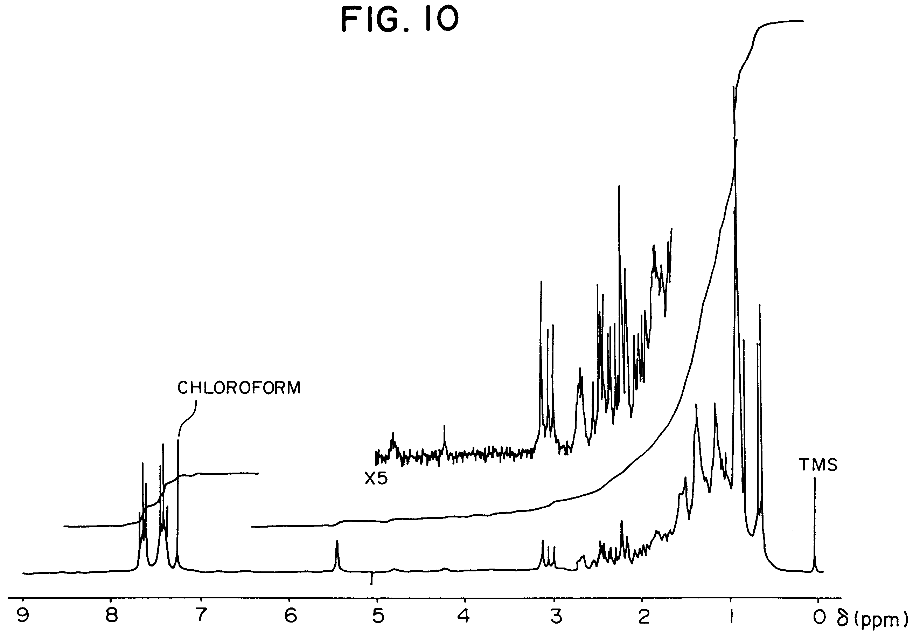

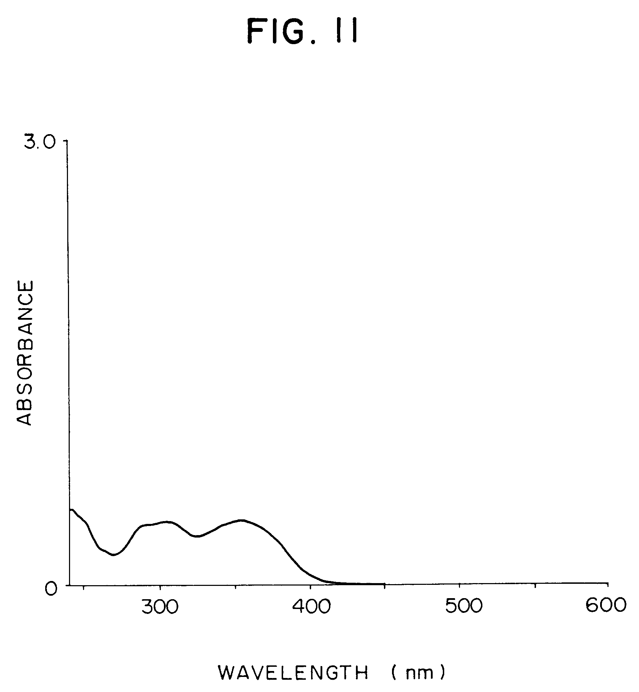

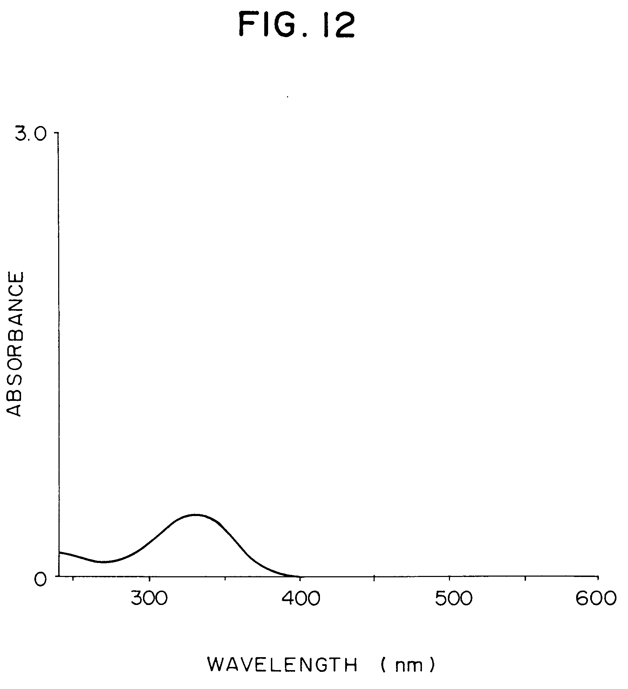

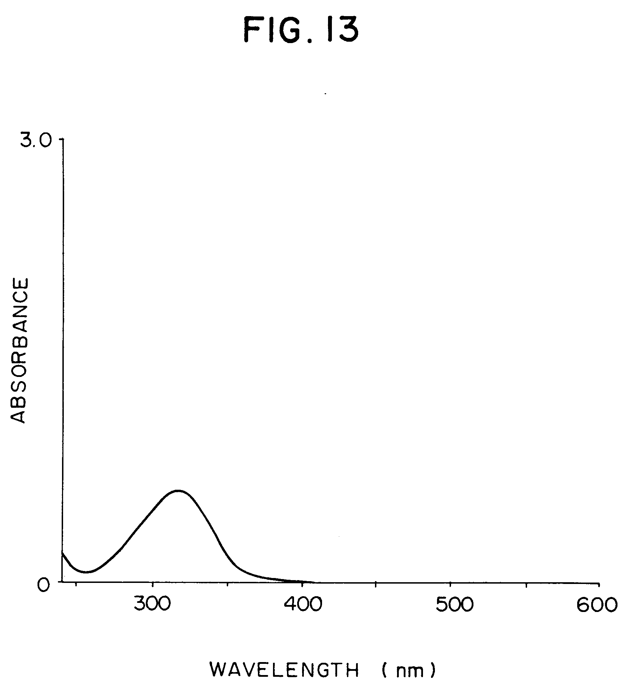

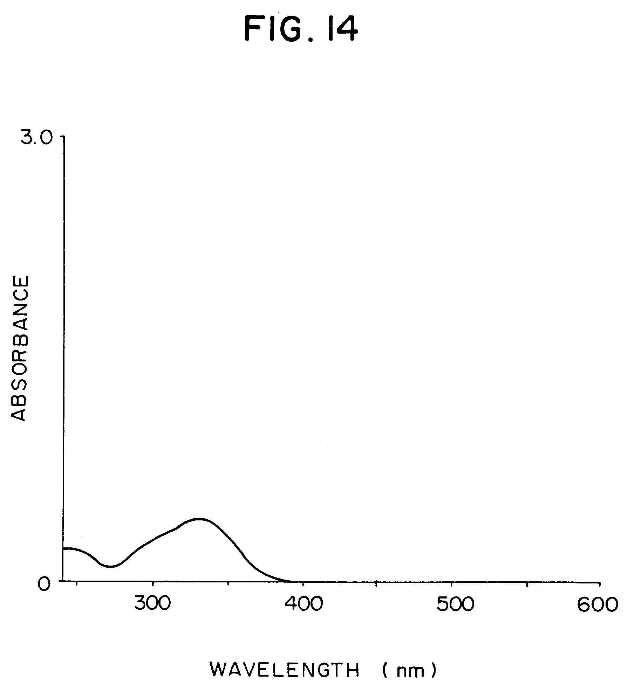

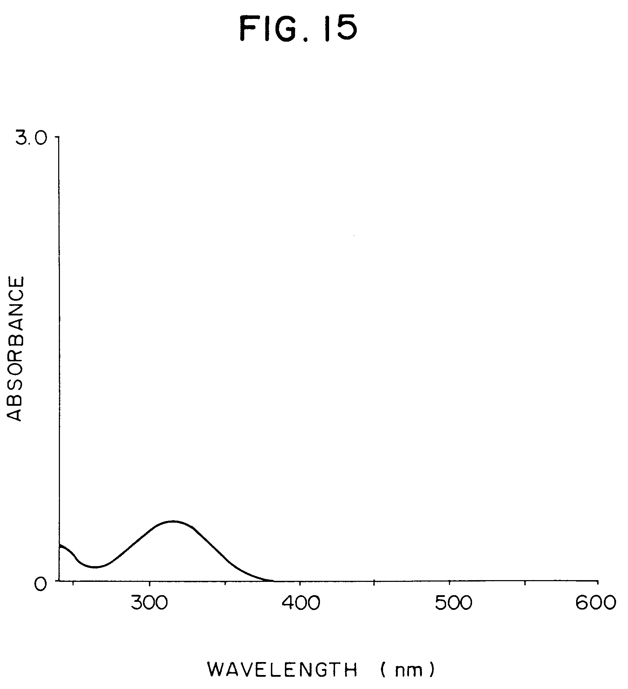

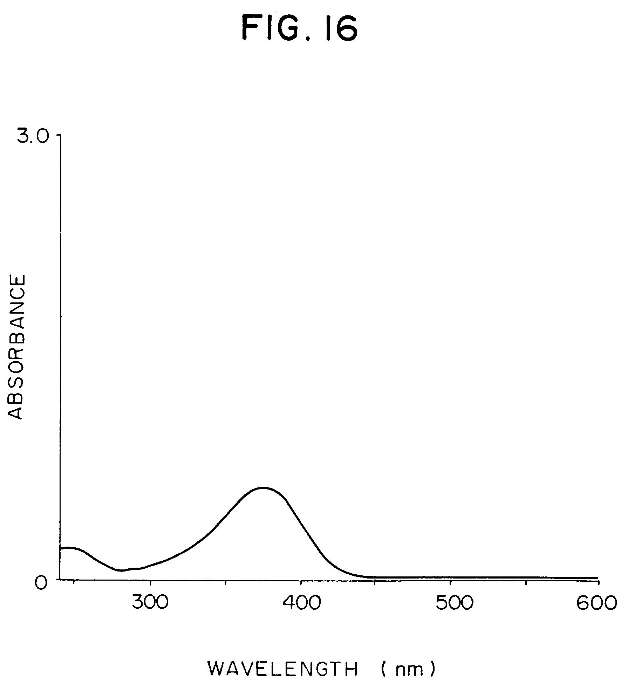

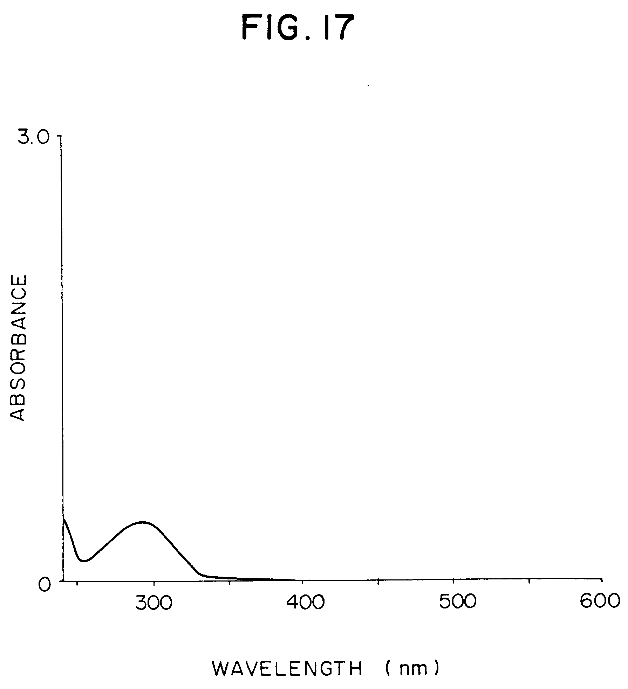

Fig. 1 shows the NMR chart of the steroidal ketone compound 1 obtained in Example 1. Fig. 2(a) shows the absorption spectrum of MNA. Fig. 2(b) shows the absorption spectrum of the steroidal ketone compound 1. Fig. 3 shows UV-VIS (ultraviolet-visible region absorption) spectrum of the steroidal ketone compound obtained in Example 1. Figs. 4 - 10 show the NMR charts of the steroidal ketone compounds 2 - 8 obtained in Examples 3 - 9. Figs. 11 - 17 show the UV-VIS spectra of these compounds.

### DESCRIPTION OF THE PREFERRED EMBODIMENTS

The steroidal ketone compound of the present invention is represented by the formula 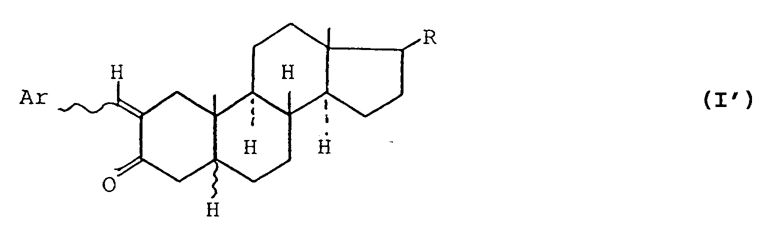
or the formula (II') 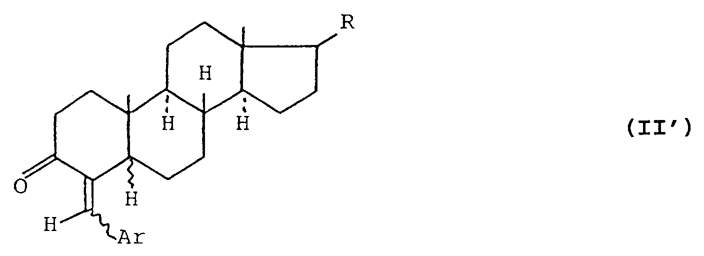
wherein R is a hydrogen atom or straight or branched alkyl group of 1 to 20 carbon atoms; Ar is a group 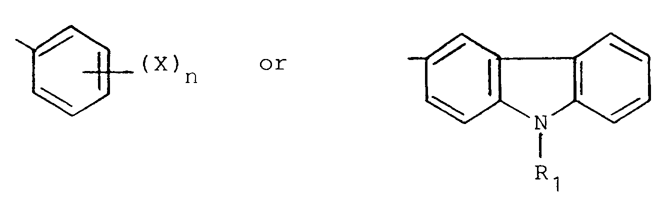
and each X is the same or different and is an amino group, an alkylamino group, a dialkylamino group, an acylated amino group, a hydroxyl group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I') or formula (II') is E or Z and the hydrogen in the 5-position has α- or β-configuration.

In the present invention, preferred is a steroidal ketone compound represented by the formula (III) or (IV): 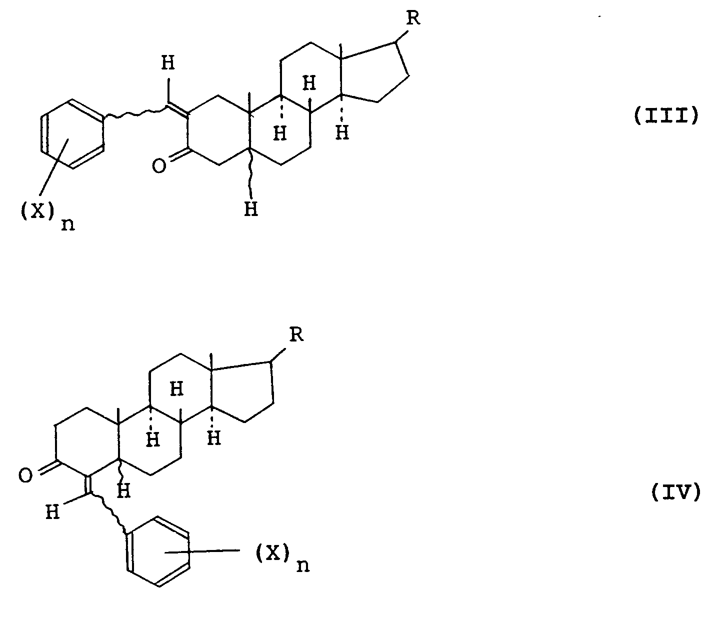
wherein R is a hydrogen atom or straight or branched alkyl group of 1 to 20 carbon atoms; each X is the same or different and is an amino group, an alkylamino group, a dialkylamino group, an acylated amino group, a hydroxyl group, a thioalkyl group, an alkyl group, a nitro group, a cyano group, or a halogen atom, the said alkyl groups and moieties being straight or branched and containing from 1 to 20 carbon atoms; n is from 1 to 5; and as denoted by the wavy lines the steroidal carbon-carbon double bond depicted in formula (III) or formula (IV) is E or Z, and the hydrogen in the 5-position has α- or β-configuration; more preferred is a compound represented by the formula: 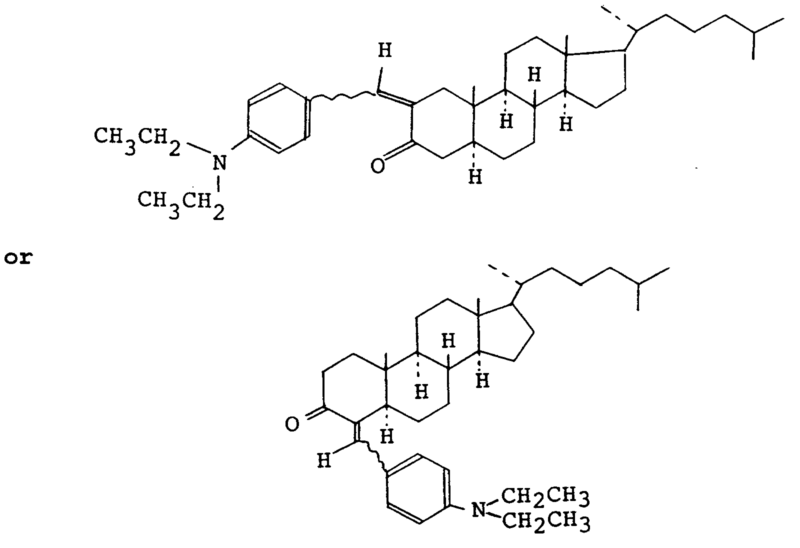
wherein the depicted steroidal carbon-carbon double bond is E or Z.

Further, the present invention provides a process for preparing the novel steroidal ketones, of formula (I') or (II') which process comprises reacting an aldehyde of the formula (V')

Ar - CHO (V')

wherein Ar is as defined in formula (I') and (II') with a steroidal compound of the formula (VI).

The invention also provides to a non-linear optical material comprising a steroidal ketone or a composition comprising a steroidal ketone, the steroidal compound being of the formula (I) 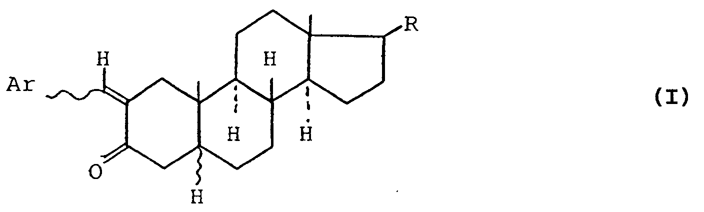
or the formula (II) 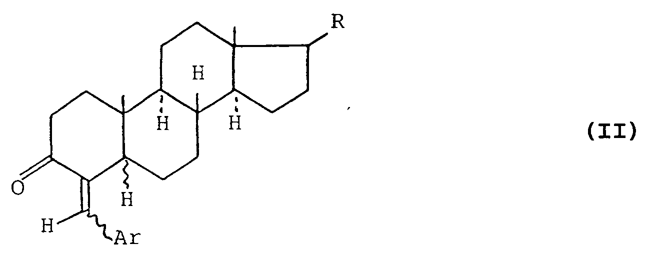
wherein R is a hydrogen atom or straight or branched alkyl group of 1 to 20 carbon atoms; Ar is a group 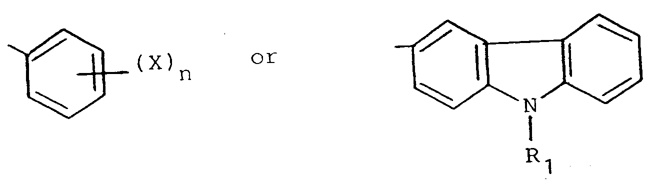
and each X is the same or different and is an amino group, an alkylamino group, a dialkylamino group, an acylated amino group, a hydroxyl group, an alkoxy group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I) or formula (II) is E or Z and the hydrogen in the 5-position has α- or β-configuration.

The invention further provides a non-linear optical device comprising a non-linear material according to the invention.

The steroidal ketone compound represented by the formula (I) or (II) (or (I') or (II')) may be obtained, through an aldol condensation described, for example, collectively in Organic Reaction Vol. 16, by reacting an aldehyde of formula (V) (or (V')) with a steroidal ketone compound of formula (VI).

Examples of the steroidal ketone compound of formula (VI) are shown below by way of their structural formulas. 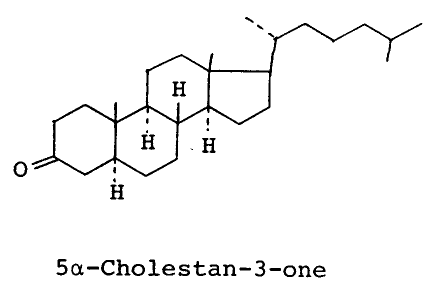 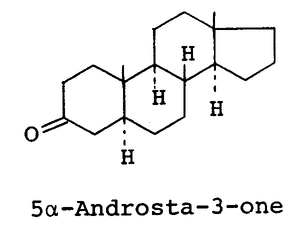
As examples of aldehydes of formula (V) or (V'), mention may be made of 4-chlorobenzaldehyde, 4-bromobenzaldehyde, 4-fluorobenzaldehyde, 4-cyanobenzaldehyde, 4-methoxybenzaldehyde, 4-methylthiobenzaldehyde, 4-aminobenzaldehyde, 4-methylaminobenzaldehyde, 4-dimethylaminobenzaldehyde, 4-ethylaminobenzaldehyde, 4-diethylaminobenzaldehyde, 4-dimethylamino-o-tolualdehyde, vanillin, o-vanillin, p-tolualdehyde, 3,4-dimethoxybenzaldehyde, 2,3,4-trimethoxybenzaldehyde, 3,4,5-trimethoxybenzaldehyde, 3-chlorobenzaldehyde, 3-bromobenzaldehyde, 3-fluorobenzaldehyde, 3-methoxybenzaldehyde, 3-methylthiobenzaldehyde, 3-aminobenzaldehyde, 3-methylaminobenzaldehyde, 3-dimethylaminobenzaldehyde, 2-methoxybenzaldehyde, and N-ethylcarbazole-3-aldehyde.

In the present invention, it is preferable to react 4-diethylaminobenzaldehyde with 5-α-cholestan-3-one.

The steroidal ketone compound of the present invention may be obtained, for example, by using an equal mol to excessive mol of the aldehyde of formula (V) or (V') relative to the steroidal ketone compound of formula (VI) (a molar ratio of the steroid compound of formula (VI) to the aldehyde in the range of 1.0 to 1.8 being preferable), and condensing the reactants in a solvent-amounting from equivalent weight to about 20 times the total weight of the steroidal ketone compound and the aldehyde in the presence of a catalyst and optionally with heating. Examples of preferred solvents include methanol, ethanol, 2-butanol, methyl Cellosolve, ethyl Cellosolve, tetrahydrofuran and dioxane. Examples of preferred catalysts include sodium hydroxide, potassium hydroxide, lithium hydroxide, barium hydroxide, tetramethylammonium hydroxide, piperidine, morpholine, sodium ethoxide and sodium methoxide. The catalyst is used in an amount of 1-5% by weight based on the weight of the steroidal ketone compound. The reaction temperature is generally from room temperature to 150°C.

The non-linear optical material according to the present invention may be obtained either by using the steroidal ketone compound mentioned above or by using a composition prepared by dissolving or dispersing the compound in a high molecular compound. Examples of high molecular compounds which may be used include homopolymers or copolymers of such monomers as methyl acrylate, methyl methacrylate, ethyl acrylate, ethyl methacrylate, n-butyl acrylate, n-butyl methacrylate, cyclohexyl acrylate, cyclohexyl methacrylate, acrylic acid, methacrylic acid, styrene, itaconic acid, maleic anhydride, vinyltoluene, divinylbenzene, vinyl chloride, β-hydroxyethyl acrylate, β-hydroxyethyl methacrylate, glycidyl acrylate and glycidyl methacrylate, polyester, polyamide, polyurethane, polycarbonate, cellulose ester, polyether, etc.

The composition mentioned above may be prepared by mixing and dissolving the steroidal ketone compound and a monomer corresponding thereto and then polymerizing the mixture with the action of light or heat, or it may be obtained by dissolving and mixing the high molecular compound mentioned above and the steroidal ketone compound through the use of a suitable solvent, followed by removal of the solvent. The non-linear optical property of the composition can be improved by performing poling during polymerization in the former method, or by performing poling after obtaining the composition both in the former method and in the latter method.

The non-linear optical material of the present invention can be used in the form of bulk crystal individually or as a part of a waveguide type optical device of fiber type, slab type, plane type, channel type, etc. Examples of the non-linear optical devices using said non-linear optical material include wavelength conversion devices which make use of second harmonic generation, sum frequency wave generation or optical parametric oscillation, and phase modulation devices and polarization plane modulation devices which make use of an electro-optical effect.

The steroidal ketone compound of the present invention has a π electron system which further increases its polarity in excited states. Moreover, the molecules of the compound have a rigid and bulky steric regulating group, so that they are, in aggregated states, apt to assume a structure of noncentrosymmetric, and hence the compound does not lose its lowest order non-linear optical effects such as second harmonic generation, Pockels effect, etc.

The present invention will be described in detail below with reference to Examples.

### Example 1

### Synthesis of steroidal ketone compound 1

In a 50-ml pear shaped flask were placed 1.33 g of 5α-cholestan-3-one and 600 mg of 4-diethylaminobenzaldehyde, then 10 ml of methanol and 2 ml of an aqueous 40% sodium hydroxide solution were added thereto, and the mixture was heated under reflux for 24 hours while stirring with a magnetic stirrer. The reaction mixture was poured into an ice-water mixture, upon which pale yellow powdery crystals separated out. The crystals were collected by filtration under suction and dried under vacuum in a desicator. The yield was 1.23 g (about 64%). The crude crystals thus obtained were purified by recrystallization from acetone. M.p. : 151.3°C.

Fig. 1 shows the NMR chart of the compound thus obtained (250 MHz, solvent used for measurement : deutero chloroform, interval standard used : tetramethylsilane (TMS)). Fig. 3 shows UV-VIS spectrum of the compound in methylene chloride. The absorption maximum wavelength was 387.2 nm.

The compound is estimated to have the following structure.

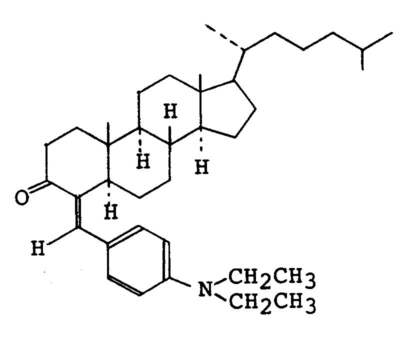

### Example 2

The steroidal ketone compound 1 synthesized in Example 1 was ground thoroughly in a mortar, and particles of a diameter of 100 µm to 125 µm were collected by use of sieves. The particles thus collected were examined for the second harmonic generation efficiency by means of the powder method using a Pulse Nd : YAG Laser (Type SL303, mfd. by Spectrolaser System Corp., output : 850 mJ, half value width : 15 ns, output per pulse : 50 MW, beam diameter : 9.5 mm, wave length : 1.064 µm). The intensity of the second harmonic generated was examined by separating the light of 532 nm with a monochromator and by using a photo-multiplier. The intensity was found to be about 0.8 time that of MNA (4-nitro-2-methylaniline), a known material.

The absorption spectrum of the crystal of the compound is shown in Fig. 2 in comparison with that of MNA. In Fig. 2, (a) refers to the absorption spectrum of MNA and (b) refers to that of the steroidal ketone compound 1. It is recognized that the α-benzylidene-steroid ketone compound 1 has a more excellent transparency in the visible region and the same second harmonic generation efficiency as compared with MNA.

### Examples 3-9

4-Diethylaminobenzaldehyde, 600 mg, used in Example 1 was replaced respectively with 756 mg of N-ethylcarbazole-3-aldehyde, 515 mg of 4-methylthiobenzaldehyde, 461 mg of 4-methoxybenzaldehyde, 563 mg of 3,4-dimethoxybenzaldehyde, 664 mg of 3,4,5-trimethoxybenzaldehyde, 505 mg of 4-dimethylaminobenzaldehyde and 444 mg of 4-cyanobenzaldehyde and reacted in the same manner as in Example 1 with 1.33 g of 5α-cholestan-3-one in methanol with sodium hydroxide used as the catalyst under reflux with heating. The reaction product was allowed to cool to form crystals. The crystals were collected by filtration under suction, washed thoroughly with cold methanol and dried under vacuum in a desicator. The resulting crude crystals were purified by recrystallization from acetone (the resulting products are respectively termed Compounds 2, 3, 4, 5, 6, 7 and 8). The melting points and UV-VIS absorption maximum wavelengths of these compounds thus obtained are shown in Table 1. Further, the respective NMR charts (250 MHz, solvent used in measurement : deutero chloroform, interval standard used : TMS) of the Compounds 2, 3, 4, 5, 6, 7 and 8 are shown in Figs. 4, 5, 6, 7, 8, 9 and 10. Further, the respective UV-VIS spectra of the Compounds 2, 3, 4, 5, 6, 7 and 8 determined in methylene chloride solutions are shown in Figs. 11, 12, 13, 14, 15, 16 and 17.

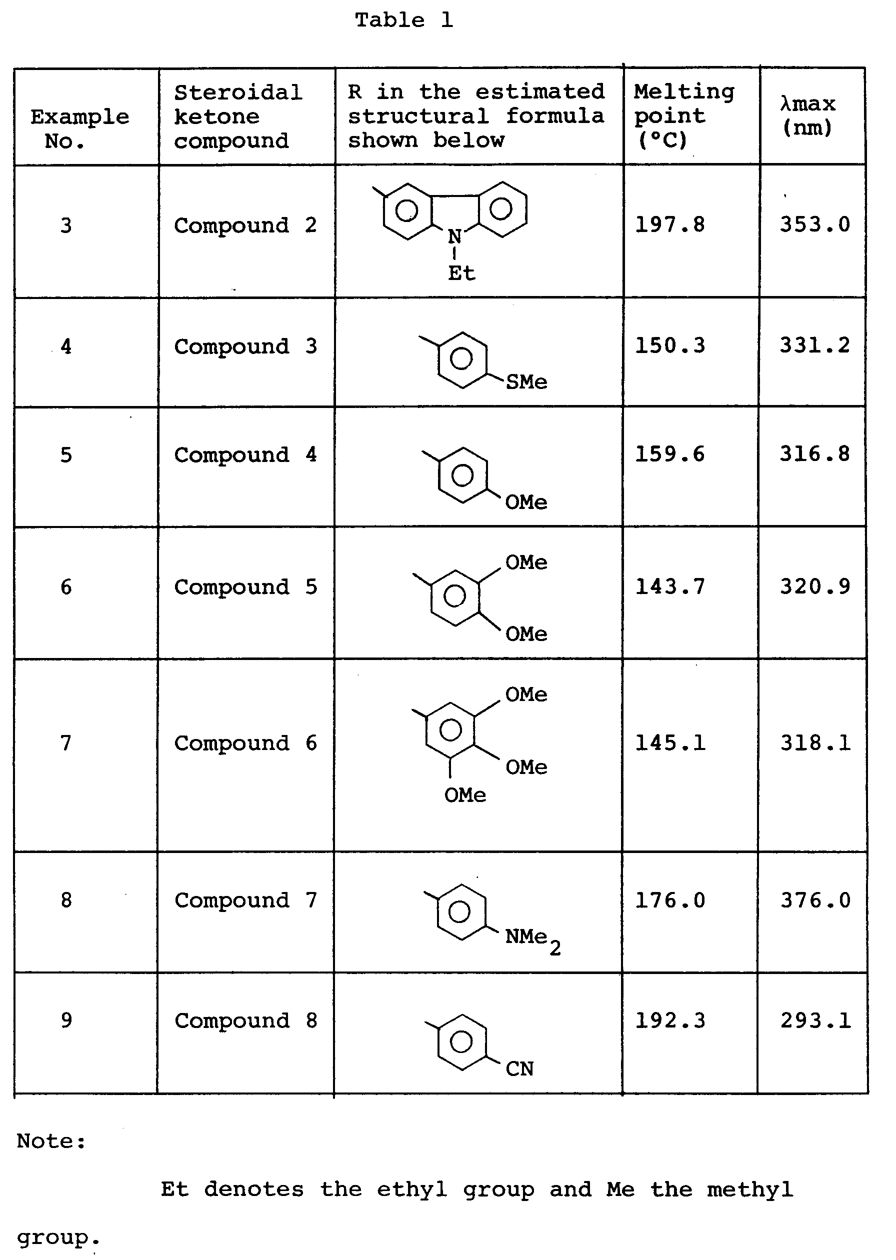

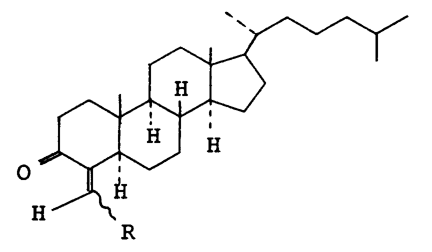

### Examples 10 - 16

The second harmonic generation (SHG) efficiencies of the Compounds 2, 3, 4, 5, 6, 7 and 8 were determined according to the same method as in Example 2, except that urea was used as the control sample in place of MNA. The results are shown in Table 2.

**Table 2**

| Example No. | Compound | SHG efficiency |
|---|---|---|
| 10 | 2 | 11 |
| 11 | 3 | 4 |
| 12 | 4 | 2.6 |
| 13 | 5 | 5 |
| 14 | 6 | 3.6 |
| 15 | 7 | 14 |
| 16 | 8 | 0.2 |
| Comparative Example | Urea | 1 |

The steroidal ketone compound of the present invention has an outstanding non-linear optical property and an excellent transparency in the visible region which is required for wavelength conversion materials and hence can provide a non-linear optical material and a non-linear optical device which have a high non-linear optical coefficient.

## Claims

1. A steroidal ketone of the formula (I') 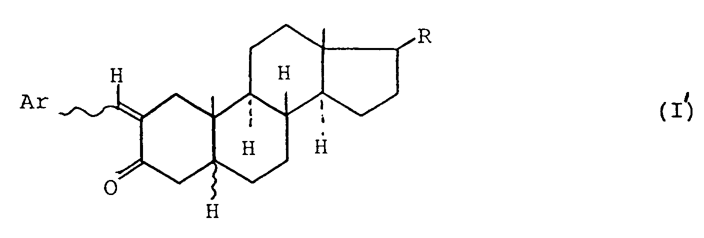 or the formula (II') 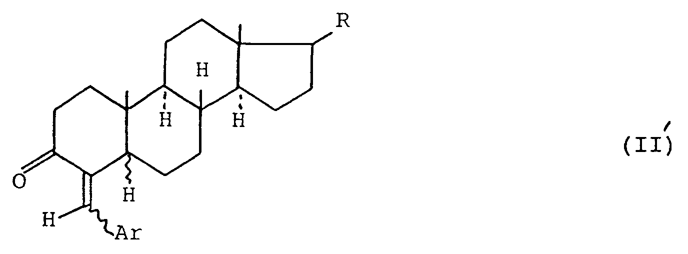 wherein R is a hydrogen atom or straight or branched alkyl group of 1 to 20 carbon atoms; Ar is a group 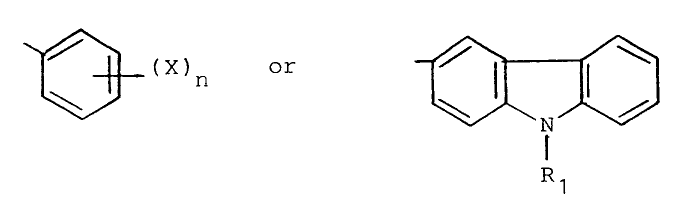 and each X is the same or different and is an amino group, an alkylamino group, a dialkylamino group, an acylated amino group, a hydroxyl group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I') or formula (II') is E or Z and the hydrogen in the 5-position has α- or β-configuration.

2. A steroidal ketone according to claim 1 of the formula (III) 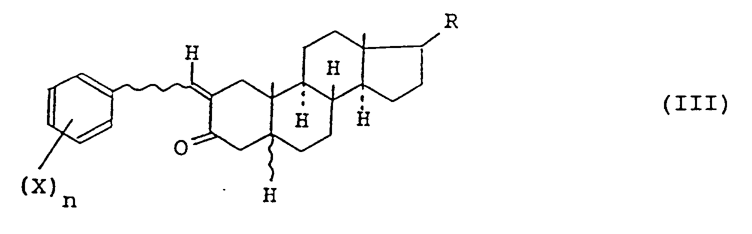 or the formula (IV) 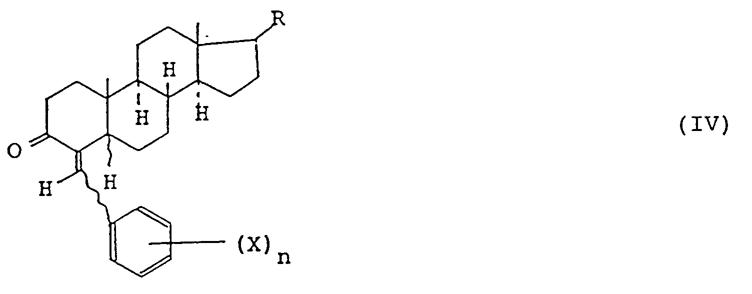 wherein R is a hydrogen atom or straight or branched alkyl group of 1 to 20 carbon atoms; each X is the same or different and is an amino group, an alkylamino group, a dialkylamino group, an acylated amino group, a hydroxyl group, a thioalkyl group, an alkyl group, a nitro group, a cyano group, or a halogen atom, the said alkyl groups and moieties being straight or branched and containing from 1 to 20 carbon atoms; n is from 1 to 5; and as denoted by the wavy lines the steroidal carbon-carbon double bond depicted in formula (III) or formula (IV) is E or Z, and the hydrogen in the 5-position has α- or β-configuration.

3. A steroidal ketone according to claim 1 of the formula: 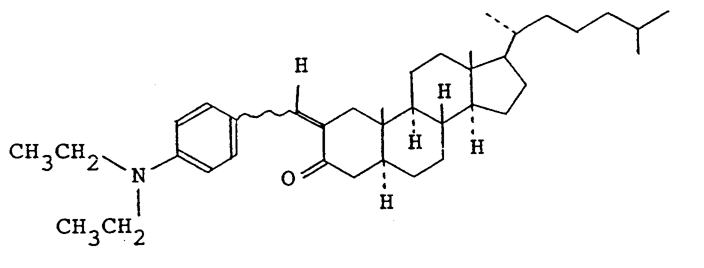 or the formula: 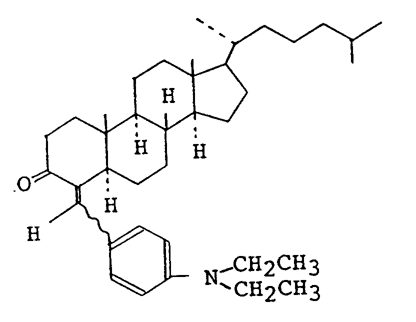 wherein the depicted steroidal carbon-carbon double bond is E or Z.

4. A process for producing a compound as claimed in claim 1 which comprises reacting an aldehyde of the formula (V')
Ar - CHO (V')
wherein Ar is as defined in claim 1 with a steroidal ketone compound of the formula (VI): 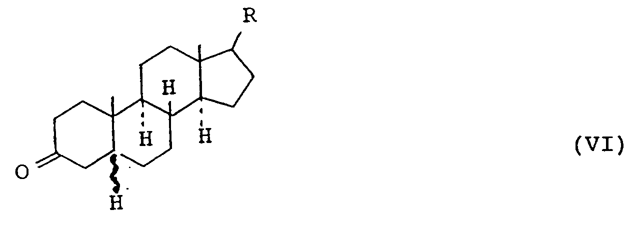 wherein R is as defined in claim 1, and the bond depicted by a wavy line is R or S.

5. A process according to claim 4 which comprises reacting 4-diethylaminobenzaldehyde with 5α-cholestan-3-one.

6. A non-linear optical material comprising a steroidal ketone of the formula (I) 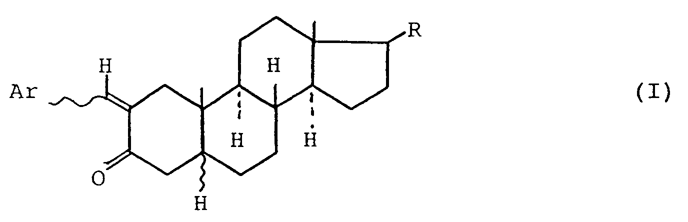 or the formula (II) 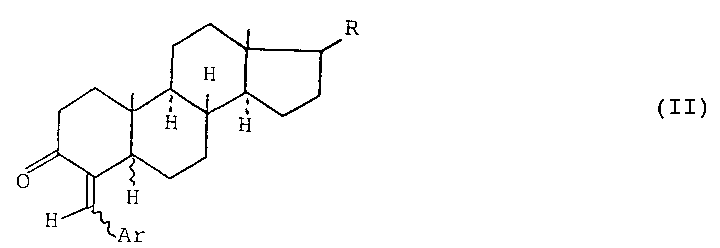 wherein R is a hydrogen atom or straight or branched alkyl group of 1 to 20 carbon atoms; Ar is a group 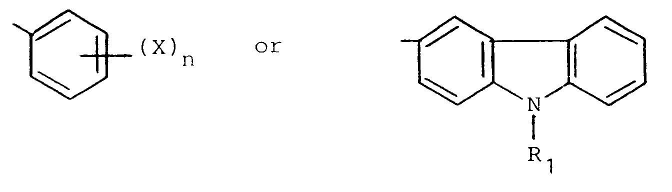 and each X is the same or different and is an amino group, an alkylamino group, a dialkylamino group, an acylated amino group, a hydroxyl group, an alkoxy group, a thioalkyl group, an alkyl group, a nitro group, a cyano group, or a halogen atom, the said alkyl groups and moieties being straight or branched and containing from 1 to 20 carbon atoms; n is from 1 to 5; R₁ is an ethyl group; and as denoted by the wavy lines the steroidal carbon-carbon double bond depicted in formula (I) or formula (II) is E or Z and the hydrogen in the 5-position has α- or β-configuration.

7. A non-linear optical material according to claim 6 wherein in the steroidal ketone Ar is a group 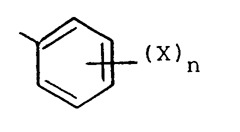 and X is the same or different and is an amino group, an alkylamino group, a dialkylamino group, an acylated amino group, a hydroxyl group, a thioalkyl group or an alkyl group.

8. A non-linear optical material according to claim 6 comprising a steroidal ketone as claimed in any one of claims 1 to 3.

9. A non-linear optical material comprising a composition which comprises a steroidal ketone as defined in any one of claims 1 to 3, 6 and 7.

10. A non-linear optical device comprising a non-linear optical material as claimed in any one of claims 6 to 9.

## Patentansprüche

1. Steroides Keton der Formel (I'): 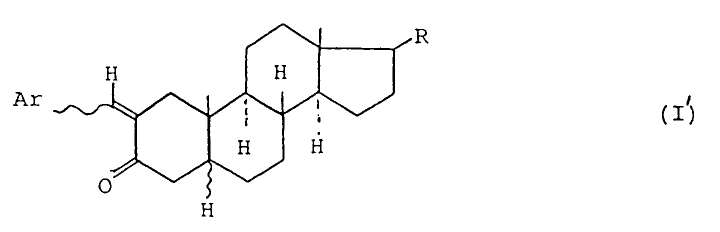 oder der Formel (II'): 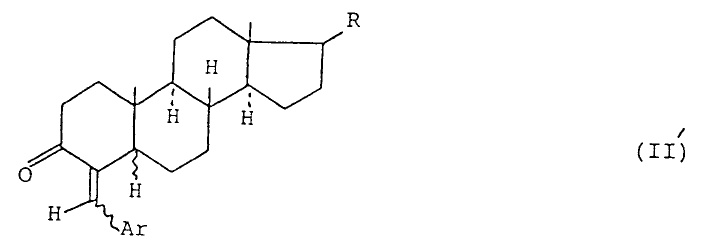 worin R für ein Wasserstoffatom oder eine gerade oder verzweigte Alkylgruppe mit 1 bis 20 Kohlenstoffatomen steht; Ar eine Gruppe 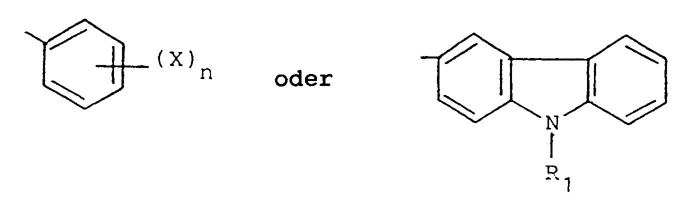 darstellt und jedes X gleich oder verschieden ist und jeweils eine Aminogruppe, eine Alkylaminogruppe, eine Dialkylaminogruppe, eine acylierte Aminogruppe, eine Hydroxylgruppe, eine Thioalkylgruppe, eine Alkylgruppe, eine Nitrogruppe, eine Cyanogruppe oder ein Halogenatom bedeutet, wobei die genannten Alkylgruppen und Gruppierungen geradkettig oder verzweigt sind und 1 bis 20 Kohlenstoffatome enthalten; n 1 bis 5 ist; R₁ für eine Ethylgruppe steht; und die durch die Wellenlinien angegebene Steroid-Kohlenstoff-Kohlenstoff-Doppelbindung in Formel (I') oder Formel (II') E oder Z ist, und wobei der Wasserstoff in 5-Position α- oder β-Konfiguration hat.

2. Steroides Keton nach Anspruch 1 der Formel (III): 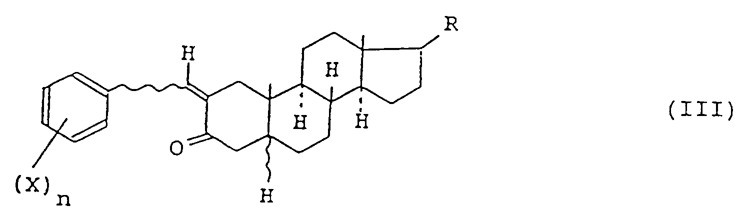 oder der Formel (IV): 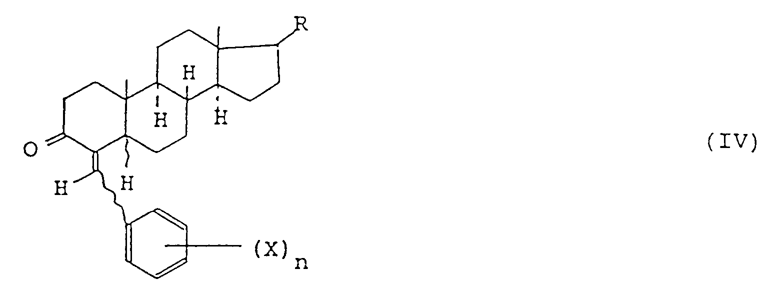 worin R für ein Wasserstoffatom oder eine gerade oder verzweigte Alkylgruppe mit 1 bis 20 Kohlenstoffatomen steht; jedes X gleich oder verschieden ist und jeweils eine Aminogruppe, eine Alkylaminogruppe, eine Dialkylaminogruppe, eine acylierte Aminogruppe, eine Hydroxylgruppe, eine Thioalkylgruppe, eine Alkylgruppe, eine Nitrogruppe, eine Cyanogruppe oder ein Halogenatom bedeutet, wobei die genannten Alkylgruppen und Gruppierungen geradkettig oder verzweigt sind und 1 bis 20 Kohlenstoffatome enthalten; n 1 bis 5 ist; und die durch die Wellenlinien angegebene Steroid-Kohlenstoff-Kohlenstoff-Doppelbindung in Formel (III) oder Formel (IV) E oder Z ist, und wobei der Wasserstoff in 5-Position α- oder β-Konfiguration hat.

3. Steroides Keton nach Anspruch 1 der Formel: 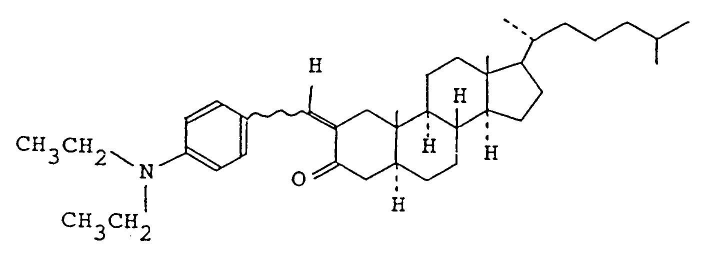 oder der Formel: 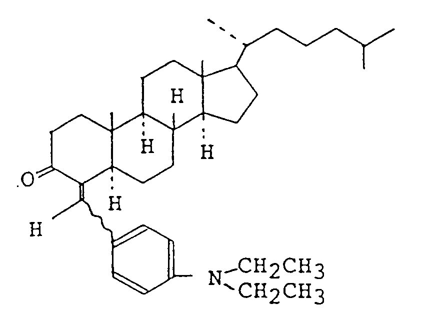 worin die angegebene Steroid-Kohlenstoff-Kohlenstoff-Doppelbindung E oder Z ist.

4. Verfahren zur Herstellung einer Verbindung nach Anspruch 1, umfassend die Umsetzung eines Aldehyds der Formel (V'):
Ar - CHO (V')
worin Ar wie in Anspruch 1 definiert ist, mit einer steroiden Ketonverbindung der Formel (VI): 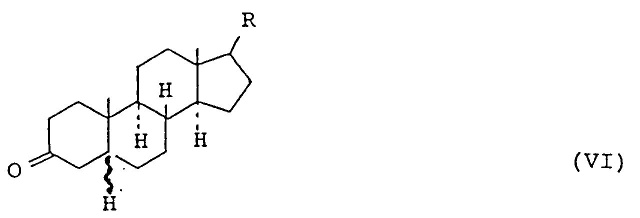 worin R wie in Anspruch 1 definiert ist und die durch die Wellenlinie angegebene Bindung R oder S ist.

5. Verfahren nach Anspruch 4, dadurch **gekennzeichnet,** daß man 4-Diethylaminobenzaldehyd mit 5α-Cholestan-3-on umsetzt.

6. Nichtlineares optisches Material, umfassend ein steroides Keton der Formel (I): 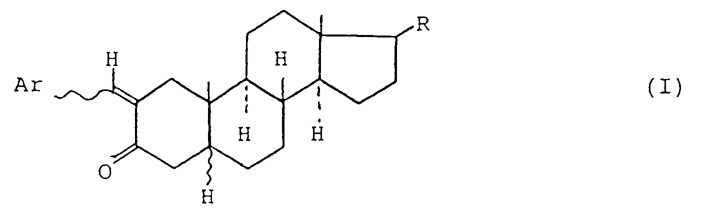 oder der Formel (II): 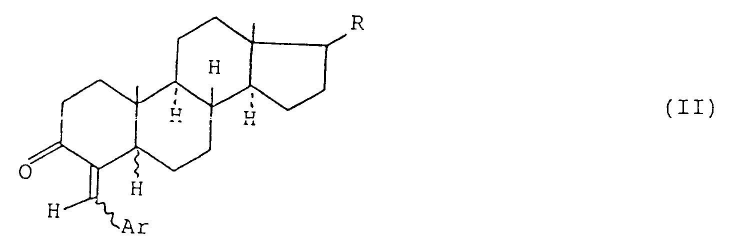 worin R für ein Wasserstoffatom oder eine gerade oder verzweigte Alkylgruppe mit 1 bis 20 Kohlenstoffatomen steht; Ar eine Gruppe 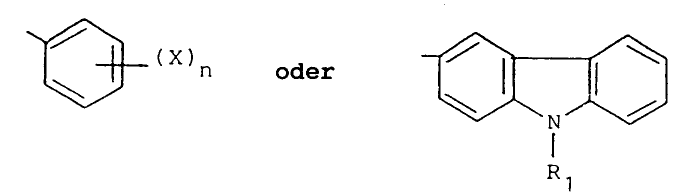 darstellt und jedes X gleich oder verschieden ist und jeweils eine Aminogruppe, eine Alkylaminogruppe, eine Dialkylaminogruppe, eine acylierte Aminogruppe, eine Hydroxylgruppe, eine Alkoxygruppe, eine Thioalkylgruppe, eine Alkylgruppe, eine Nitrogruppe, eine Cyanogruppe oder ein Halogenatom bedeutet, wobei die genannten Alkylgruppen und Gruppierungen geradkettig oder verzweigt sind und 1 bis 20 Kohlenstoffatome enthalten; n 1 bis 5 ist; R₁ für eine Ethylgruppe steht; und die durch die Wellenlinien angegebene Steroid-Kohlenstoff-Kohlenstoff-Doppelbindung in Formel (I) oder Formel (II) E oder Z ist und wobei der Wasserstoff in 5-Position α- oder β-Konfiguration hat.

7. Nichtlineares optisches Material nach Anspruch 6, dadurch **gekennzeichnet,** daß die Gruppierung Ar des steroiden Ketons eine Gruppe: 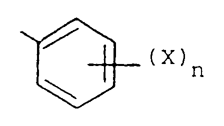 ist und wobei X gleich oder verschieden ist und für eine Aminogruppe, eine Alkylaminogruppe, eine Dialkylaminogruppe, eine acylierte Aminogruppe, eine Hydroxylgruppe, eine Thioalkylgruppe oder eine Alkylgruppe steht.

8. Nichtlineares optisches Material nach Anspruch 6, dadurch **gekennzeichnet,** daß es ein steroides Keton nach einem der Ansprüche 1 bis 3 enthält.

9. Nichtlineares optisches Material, umfassend eine Zusammensetzung, umfassend ein steroides Keton nach einem der Ansprüche 1 bis 3, 6 und 7.

10. Nichtlineare optische Vorrichtung, umfassend ein nichtlineares optisches Material nach einem der Ansprüche 6 bis 9.

## Revendications

1. Cétone stéroïde de formule (I') 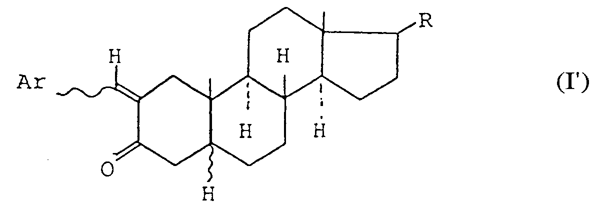 ou de formule (II') 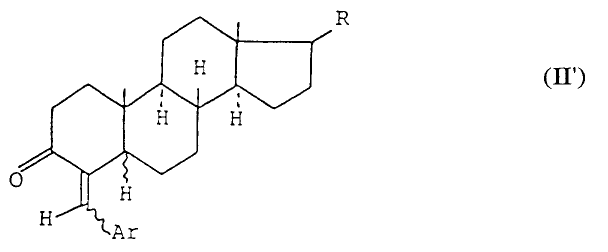 dans lesquelles formules R représente un atome d'hydrogène ou un groupe alkyle linéaire ou ramifié comportant de 1 à 20 atomes de carbone ; Ar représente un groupe de formule 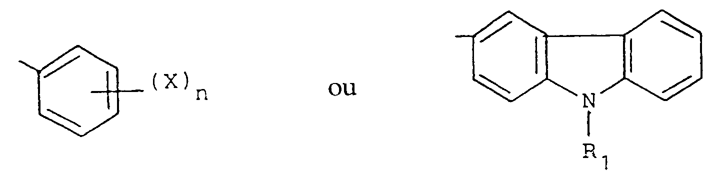 où les substituants X sont identiques ou différents et sont des groupes amino, alkylamino, dialkylamino, acylamino, hydroxy, alkylthio, alkyle, nitro ou cyano ou des atomes d'halogène, lesdits groupes ou fragments alkyle étant linéaires ou ramifiés et comportant de 1 à 20 atomes de carbone, n vaut de 1 à 5 et R₁ représente un groupe éthyle ; et les traits ondulés indiquent que la double liaison carbone-carbone concernée du stéroïde de formule (I') ou (II') a une configuration E ou Z et que l'atome d'hydrogène placé en position n° 5 se trouve en configuration α ou β.

2. Cétone stéroïde conforme à la revendication 1, de formule (III) 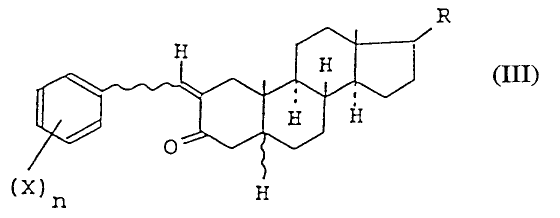 ou de formule (IV) 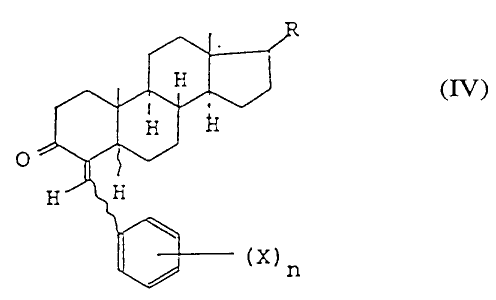 dans lesquelles formules R représente un atome d'hydrogène ou un groupe alkyle linéaire ou ramifié comportant de 1 à 20 atomes de carbone, les substituants X sont identiques ou différents et sont des groupes amino, alkylamino, dialkylamino, acylamino, hydroxy, alkylthio, alkyle, nitro ou cyano ou des atomes d'halogène, lesdits groupes ou fragments alkyle étant linéaires ou ramifiés et comportant de 1 à 20 atomes de carbone, n vaut de 1 à 5 et les traits ondulés indiquent que la double liaison carbone-carbone concernée du stéroïde de formule (III) ou (IV) a une configuration E ou Z et que l'atome d'hydrogène placé en position n° 5 se trouve en configuration α ou β.

3. Cétone stéroïde conforme à la revendication 1, de formule 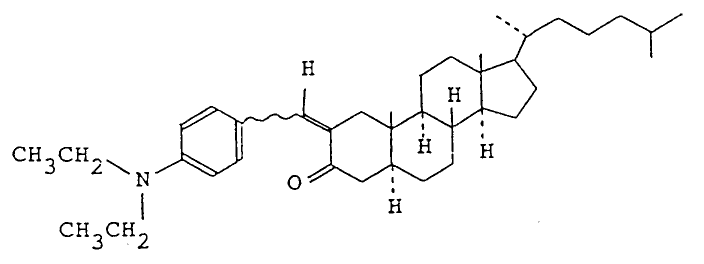 ou de formule 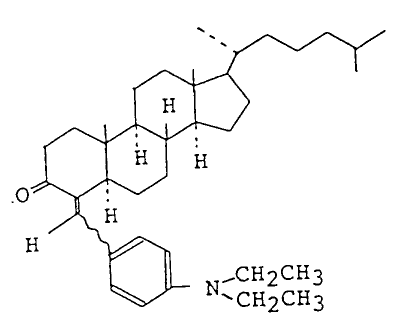 où le trait ondulé indique que la double liaison carbone-carbone concernée du stéroïde a une configuration E ou Z.

4. Procédé de préparation d'un composé conforme à la revendication 1, qui comporte le fait de faire réagir un aldéhyde de formule (V')
Ar-CHO (V')
dans laquelle Ar a la même signification que dans la revendication 1, avec une cétone stéroïde de formule (VI) 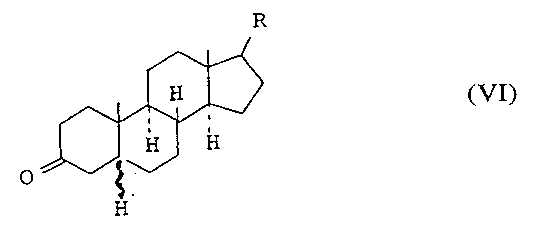 dans laquelle R a la même signification que dans la revendication 1, et la liaison indiquée par un trait ondulé correspond à une configuration R ou S.

5. Procédé conforme à la revendication 4, qui comporte le fait de faire réagir du 4-diéthylamino-benzaldéhyde avec de la 5α-cholestane-3-one.

6. Matériau à réponse optique non linéaire, comprenant une cétone stéroïde de formule (I) 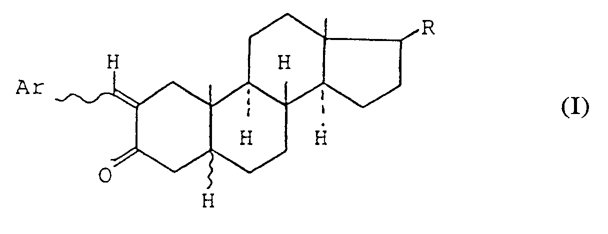 ou de formule (II) 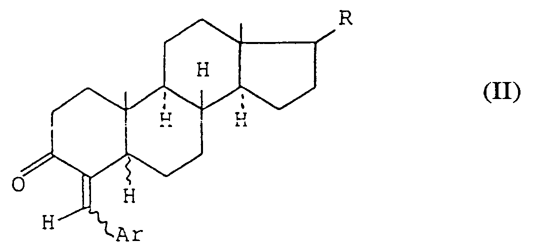 dans lesquelles formules R représente un atome d'hydrogène ou un groupe alkyle linéaire ou ramifié comportant de 1 à 20 atomes de carbone ; Ar représente un groupe de formule 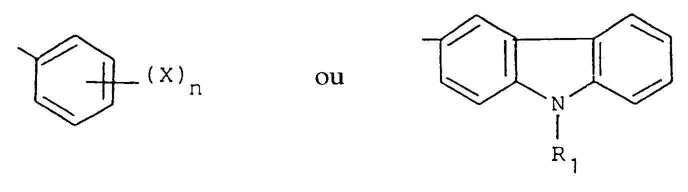 où les substituants X sont identiques ou différents et sont des groupes amino, alkylamino, dialkylamino, acylamino, hydroxy, alcoxy, alkylthio, alkyle, nitro ou cyano ou des atomes d'halogène, lesdits groupes ou fragments alkyle étant linéaires ou ramifiés et comportant de 1 à 20 atomes de carbone, n vaut de 1 à 5 et R₁ représente un groupe éthyle ; et les traits ondulés indiquent que la double liaison carbone-carbone concernée du stéroïde de formule (I) ou (II) a une configuration E ou Z et que l'atome d'hydrogène placé en position n° 5 se trouve en configuration α ou β.

7. Matériau à réponse optique non linéaire, conforme à la revendication 6, dans lequel, dans la cétone stéroïde, Ar représente un groupe de formule 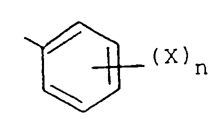 où les substituants X sont identiques ou différents et sont des groupes amino, alkylamino, dialkylamino, acylamino, hydroxy, alkylthio ou alkyle.

8. Matériau à réponse optique non linéaire, conforme à la revendication 6, comprenant une cétone stéroïde conforme à l'une des revendications 1 à 3.

9. Matériau à réponse optique non linéaire, comprenant une composition qui contient une cétone stéroïde définie dans l'une des revendications 1 à 3, 6 et 7.

10. Dispositif à réponse optique non linéaire, comprenant un matériau à réponse optique non linéaire conforme à l'une des revendications 6 à 9,
